# EUROPEAN PATENT SPECIFICATION

(11) **EP 2 468 357 B1**
(45) Date of publication and mention of the grant of the patent: **28.10.2015**
(21) Application number: 10196637.2
(22) Date of filing: 22.12.2010
(51) Int. Cl.: A61N 1/362, A61B 5/00

(54) **Implantable medical device**
Implantierbare medizinische Vorrichtung
Dispositif médical implantable

(43) Date of publication of application: 27.06.2012
(73) Proprietor: St. Jude Medical AB, 175 84 Järfälla (SE)
(72) Inventor: Hollmark, Malin, 169 73 Solna (SE); Karlsson, Andreas, 169 67 Solna (SE)
(74) Representative: Sjölander, Henrik

(56) References cited:
- EP-A1- 1 384 433
- WO-A1-2004/008959
- WO-A1-2004/028629
- WO-A1-2009/096820

## Description

### Field of the invention

The present invention relates to a device and a method according to the preambles of the independent claims, and in particular to a device and a method adapted to determine a risk of atrial fibrillation.

### Background of the invention

Atrial fibrillation is a very common arrhythmia. During episodes of atrial fibrillation, the systolic function of the atria is lost. This results in dilatation of the atria which in turn makes it more difficult for the heart to return to sinus rhythm. Without regular systolic activity the atria will only be passive mediators of blood volume to the ventricles. The degree of dilatation of the atria will reflect the venous return, i.e. preload.

Recent experimental animal studies have demonstrated that the right atrial (RA) stretch and dilatation can lead to development of atrial fibrillation (AF) (Ravelli 2003, Mechano-Electric Feedback and Cardiac Arrhythmias, Progress in Biophysics and Molecular Biology, 82(1-3)137-149). In addition, RA dilatation follows left atrial (LA) dilatation and vice versa. Thus, monitoring of the volume of one atrium will provide monitoring of the other.

WO-2004/028629 relates to a heart stimulator detecting atrial arrhythmia by determining wall distension by impedance measurement. Upon detection of an atrial arrhythmia the stimulation mode is switched and the pacing rate is adapted to limit the atrial distension. The heart stimulator may also be used for monitoring the degree of atrial distension over an extended period of time to be able to follow the disease development and to enable the physician to adapt therapy accordingly.

In a research paper ("Effects of spironolactone on atrial structural remodelling in a canine model of atrial fibrillation produced by prolonged atrial pacing", J Zhao et al, British Journal of Pharmacology (2010), 159, pp 1584-1594) it is briefly discussed the generally accepted fact that atrial fibrillation (AF) and atrial dilatation may be mutually dependent and constitute a vicious circle. LA dilatation has been identified as an independent risk factor for the development of AF. AF results in progressive atrial dilatation, which in turn, might contribute to the self-perpetuating nature of arrhythmia. Atrial dilatation is due to an increase in atrial compliance caused by atrial contractile dysfunction during AF. An increase in atrial size will facilitate the development of atrial fibrillation. Furthermore, an elevated intra-atrial pressure will increase atrial wall stress, which may result in heterogeneities in conduction. In addition, atrial dilatation may promote focal arrhythmias that trigger self-perpetuating AF or maintain irregular atrial activation by mechano-electric feedback. The increased inhomogenity in atrial electrophysiological properties during atrial dilatation contributes to the development of AF. According to the presented data, interventions targeting a reduction of LA size may prevent AF or AF disease progression. The inventors have identified the relationship between an increased atrial dilatation and the risk of developing atrial fibrillation, and the object of the present invention is to achieve an improved device and method of determining atrial dilatation and, thus, the risk of developing atrial fibrillation.

WO 2009/096820 uses correlated measured for predicting potential occurrence of AF. An impedance of a patient is measured and used to obtain cardiogenic data and respiratory data. A hemodynamic measure is calculated from the cardiogenic data and an apnea measure is calculated from the respiratory data. The measures are correlated and used to predict AF occurrence.

### Summary of the invention

The above-mentioned object is achieved by the present invention according to the independent claims.

Preferred embodiments are set forth in the dependent claims.

Thus, the inventors have found that by using so-called near-field immittance measurements, local measurement values may be determined being specifically suitable for determining a measure of atrial dilatation.

Using the proposed approach the present invention aims at monitoring the heart chamber volumes using impedance. This enables dilatation monitoring and, ultimately, AF or AF disease progression prevention. Early dilatation detection prior to AF can deter the disease progression by early medical intervention.

In a dilated heart, the blood volume in the proximity of an electrode is larger and varies less during the heart cycle than in a normal healthy heart. Such differences between a dilated chamber and a healthy chamber can be detected by measuring and analysing the impedance signal from the chamber in question. Chamber dilatation is detected as a decreased average impedance as well as lower peak-to-peak variation of the impedance.

Thus, a recorded impedance waveform reflects the superposition of fluid volume around the electrode pair throughout the cardiac and respiratory cycles. As an example, when measuring e.g. the impedance between an RAring electrode (right atrial ring electrode) and a SVC (superior vena cava) electrode, it is possible to generate an algorithm for the calculation of fluid volume surrounding the RAring (the so-called RAring near-field), which in turn reflects the RA volume. Thus, impedance measurements associated with the RAring electrode reflects the RA volume. Since RA dilatation follows LA dilatation, monitoring of the RA volume will also provide a monitor of the LA volume.

Left-sided heart diseases often lead to increased left atrial (LA) pressure, which inevitably will lead to dilatation of LA and subsequently atrial fibrillation (AF). Early dilatation detection prior to AF will help pacemaker and CRT (cardiac resynchronization therapy) patients by deterring the disease progression through early medical intervention. The present invention suggests ambulatory monitoring of the right atrial (RA) volume by impedance measurements. Since RA dilatation follows LA dilatation, monitoring of the RA volume will also provide a monitor of the LA volume. Impedance measurement of the fluid displacement in the immediate volume surrounding the RAring will provide a measure of dilatation.

In addition to provide an AF risk indication the present invention also may provide measures to monitor the progression of AF.

### List of abbreviations

- RA: right atrium
- LA: left atrium
- RV: right ventricle
- LV: left ventricle
- RAring/RAring electrode: right atrial ring electrode
- RAtip/RAtip electrode: right atrial tip electrode
- LAring/LAring electrode: left atrial ring electrode
- LAtip/LAtip electrode: left atrial tip electrode
- RVring/RVring electrode: right ventricular ring electrode
- RVtip/RVtip electrode: right ventricular tip electrode
- LVring/LVring electrode: left ventricular ring electrode
- LVtip/LVtip electrode: left ventricular tip electrode
- RV coil: right ventricular coil electrode
- SVCoil: superior vena cava coil electrode

### Short description of the appended drawings

Figure 1 is a schematic block diagram illustrating an implantable medical device according to the present invention.
Figure 2 illustrates the principle for calculation of near-field immittance values.
Figure 3 is a flow-diagram illustrating a method according to the present invention.
Figures 4 and 5 illustrate two different electrode set-ups which both would be applicable in relation to the present invention.
Figure 6 shows a graph illustrating schematic impedance signals.
Figure 7 shows a graph illustrating impedance signals from a pre-clinical study.

### Detailed description of preferred embodiments of the invention

State-of-the art implantable medical devices are often equipped to measure impedance (or related electrical parameters such as admittance) between various pairs of electrodes implanted within the patient. Examples include intracardiac impedance measurements made between pairs of electrodes mounted to leads implanted on or within the various chambers of the heart. Other examples include intrathoracic impedance measurements made between the housing of the device (or "can" or "case") and electrodes implanted on or within the heart. Traditionally, such impedance measurements were deemed to be representative of the electrical impedance between the electrodes. That is, impedance measurements were associated with a particular pair of electrodes or some combination of three or more electrodes. Herein, these measurements are generally referred to as normal impedance measurements, or only "impedance measurements", because the measurements are associated with at least one pair of electrodes. In terms of analyzing and interpreting the measured impedance data, the interpretation typically relied on a conceptual model wherein the measured impedance was deemed to be representative of the impedance of the field between the electrode pairs, including far-field contributions to that impedance. Under the far-field model, impedance measured between a pair of electrodes A and B is deemed to be representative of the field between A and B.

As one example of the far-field model, intrathoracic impedance measurements made between the device housing and a cardiac electrode implanted within the heart are deemed to represent the impedance to electrical flow spanning a field extending through the lungs between the device and the cardiac electrode. This intrathoracic impedance measurement may then be used to, for example, assess pulmonary fluid congestion to detect pulmonary oedema (PE) or heart failure (HF). Although this traditional interpretation of the impedance measurements can be useful, it has been recognized that an alternative interpretation of impedance measurements based on a "near-field model" can provide a more useful means for understanding, analyzing and interpreting impedance measurements.

The present invention is generally directed to the near-field impedance model and various systems, methods and applications that exploit this model.

In accordance with exemplary embodiments of the invention, an implantable medical device, such as a pacemaker, an implantable cardioverter defibrillator (ICD) or a cardiac resynchronization therapy (CRT) device, and a method for use in an implantable medical device, are provided for determining and exploiting near-field immittance values (wherein "immittance" broadly refers to impedance, conductance, admittance or other generally equivalent electrical values or parameters) associated with individual electrodes in accordance with a near-field model that associates immittance values with individual electrodes rather than with pairs of electrodes.

In this regard, exemplary techniques provided herein exploit the aforementioned near-field model, which offers a new perspective for the interpretation of the immittance measurements that significantly simplifies the analysis and interpretation of data and the development of detection methods/procedures. Briefly, the near-field model is based on the recognition that the immittance between a pair of electrodes (A and B) can be modelled as a superposition of near-field immittance values that are associated with the individual electrodes (i.e. A+B). That is:

| | |
|---|---|
| Traditional model: | Immittance = A to B = Field between A and B |
| Near-field model: | Immittance = A + B = Near-field A+Near-field B |

More generally, the near-field model transforms multiple pair-based immittance measurement values into a set of near-field immittance values that can be interpreted and analyzed more easily. In an example where impedance is measured, the conversion of normal impedance measurement values into near-field impedance values is performed by converting N (where N is at least three) impedance measurement values (v1, v2, ... , vN) into a set of linear equations to be solved whereby far-field contributions to impedance are ignored. The set of linear equations are then solved to yield a set of near-field impedance values (e1, e2, ..., eN) associated with the individual electrodes. In other examples, N+1 impedance measurements (or some even larger number) are used to determine the near-field impedance values of the N electrodes.

One important advantage of the near-field model is that by deriving near-field immittance values associated with individual electrodes, the device can easily associate a specific physical entity - such as the particular anatomical structure adjacent to the electrode - with the corresponding near-field immittance value.

For example, for an RAring electrode, the corresponding near-field immittance is associated with the local fluid and tissue content within the adjacent RA cavity and RA tissues.

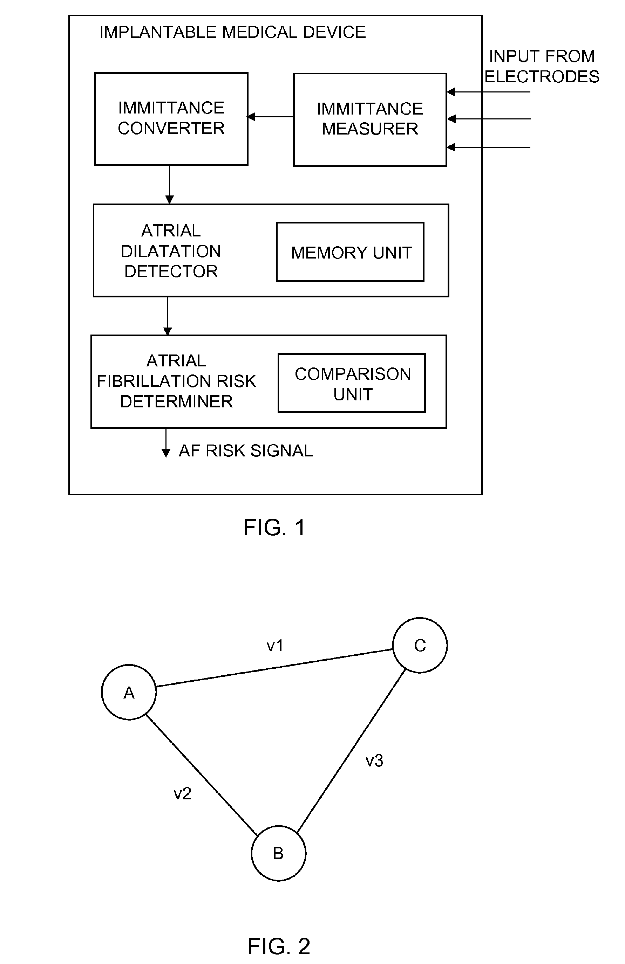

The basis of the algorithm used in the present invention is that when several immittance configurations are measured, each current node reflects the tissue-to-blood proportionality in its immediate surrounding. As an example of this, the following configuration (with reference to figure 2) may be used:
v1: RAring (shown as A in figure 2) - case (C)
v2: RAring (A) - LVring (B)
v3: LVring (B) - case (C)

These equations form an "impedance triangle" which may be solved for each node by the following equation systems:
System 1:
   v1=A+C
   v2=A+B
   v3=B+C
System 2:
   v1+v2-v3=2A=2 RAring
   v2+v3-v1=2B=2 LVring
   v1+v3-v2=2C=2 case

Thus, the three impedance waveforms measured with the three impedance fields are in fact composites made up of the three distinct waveforms from each of the three nodes, A, B and C. The three distinct waveforms are extracted by using the equation system 2 above.

Consequently, measurement of the impedances suggested above (or any other impedances that include the RAring and/or RAtip), but still creating an "impedance triangle", will provide an estimation of the fluid volume surrounding the RAring or RAtip. This, in turn, reflects the RA volume.

When performing immittance measurements bipolar configurations are not a prerequisite, quadrupolar configurations may be used as well.

For instance, the quadrupolar configuration: *I*: RAring-LVring, *U*: RAtip-LVtip could replace the bipolar configuration *I*: RAring-LVring, *U*: RAring-LVring (where *I* denotes the current injection nodes and *U* the voltage nodes).

Additionally, and within the scope of the present invention as defined by the claims, it is possible to measure impedance (immittance) over more than three anatomical locations. For example, the measurements may be performed by using geometries with four poles (e.g. RAring/RAtip, LAring(s)/LAtip, RVring/RVtip and Case). This would provide mean impedance values for the electrodes in the measured configurations.

If one specific electrode would be of special interest, this electrode may be measured against two larger electrodes. The surface ratio together with the near-field model evaluation would then ensure that the electrode of specific interest would have a significant signal contribution. The triangle could then e.g. include the following configurations: RAring-Case, RAring-RVcoil, RVcoil-Case; where the RAring-electrode is of particular interest.

The present invention will now be described in more detail with references to the block diagram shown in figure 1.
In figure 1 is schematically shown an implantable medical device according to the invention. The implantable medical device is connectable to at least three electrodes. The electrodes, to which the implantable medical device is connectable, may, for example, be selected from the group of: the case (or can) of the implantable medical device, RAring electrodes, an RAtip electrode, LAring electrodes, an LAtip electrode, LVring electrodes, an LVtip electrode, RVring electrodes, an RVtip electrode, RVcoil electrodes or SVCoil electrodes. In figure 1 the input signals from the electrodes are indicated by three parallel arrows. The implantable medical device comprises an immittance measurer operative to perform immittance measurements within the heart of a patient using at least three of said electrodes where at least one of the electrodes is arranged in an atrium of the patient's heart.

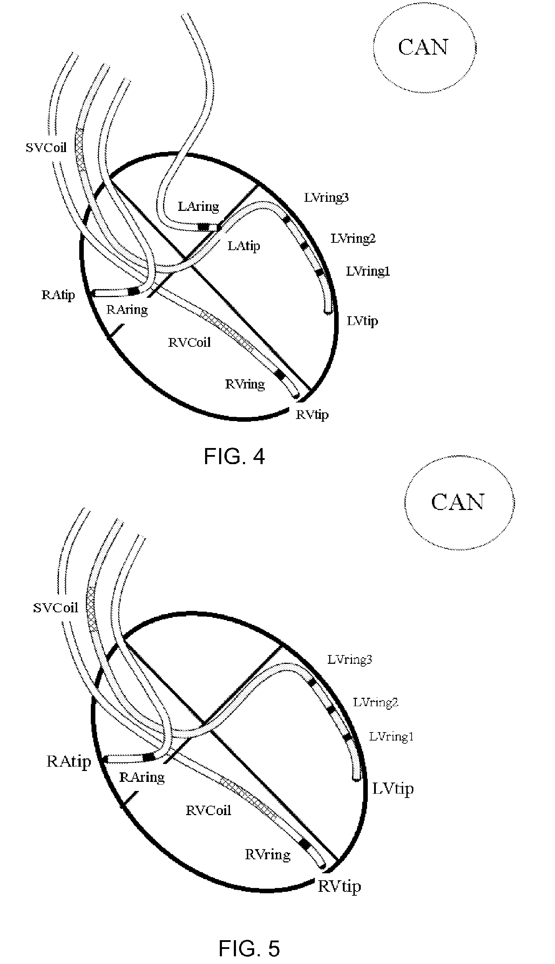

Figures 4 and 5 illustrate two different electrode set-ups which both would be applicable in relation to the present invention.

The medical device further comprises an immittance converter operative to convert immittance measurement values into individual near-field immittance values of at least one of the at least one electrode being arranged in an atrium. The device in addition comprises an atrial dilatation detector operative to detect atrial dilatation based upon the individual near-field immittance values, and to determine atrial dilatation values in dependence thereto. An atrial fibrillation risk determiner is also included in the device, which risk determiner is adapted to determine an atrial fibrillation risk index based upon the atrial dilatation values.

Preferably, the atrial fibrillation risk determiner is adapted to generate an atrial fibrillation risk signal in dependence of the risk index.

The atrial dilatation detector may also comprise a memory unit for storage of the determined atrial dilatation values.

Furthermore, the atrial fibrillation risk determiner may comprise a comparison unit provided with at least one atrial fibrillation risk threshold. The comparison unit is adapted to compare the determined atrial dilatation values with the at least one fibrillation risk threshold and the atrial fibrillation risk determiner is adapted to determine the atrial fibrillation risk index in dependence of the comparison. The atrial fibrillation risk threshold is an atrial dilatation value for which the risk of atrial fibrillation is considered to be significant.

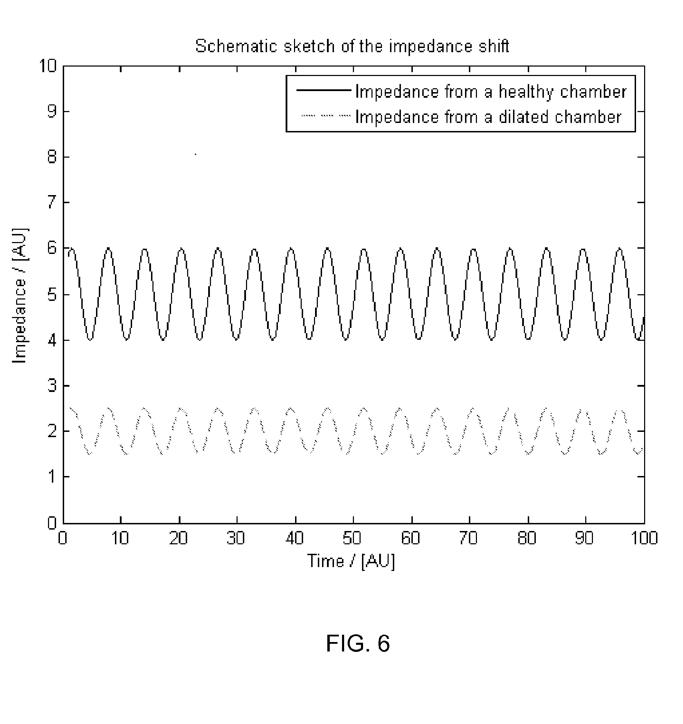

Returning to the graphs shown in figure 6 where the upper curve shows the impedance from a healthy heart chamber and the lower curve shows the impedance from a dilated heart chamber, e.g. from a right atrial ring electrode. Two significant differences between the curves may be observed. One difference is the DC-level, which is higher for the healthy heart and which is related to the smaller volume of the heart chamber. The DC-level is the average of the measured impedance. Another difference is the AC-amplitude, which is smaller for the dilated chamber than for the healthy heart chamber. This is caused by the inelasticity of the heart wall during AF. The AC-amplitude is the peak-to-peak variation of the impedance. These two parameters, the DC-level and the AC-amplitude, are advantageously used as parameters for the atrial fibrillation risk thresholds.

Preferably, the determined atrial fibrillation risk index is based upon the variations of the determined atrial dilatation values during a preset time period. The atrial dilatation variation during healthy periods can also be considered when setting the thresholds for what is to be considered a pathological change of the atrial dilatation. Thus, the risk index may be based upon the variations of the determined atrial dilatiation values.

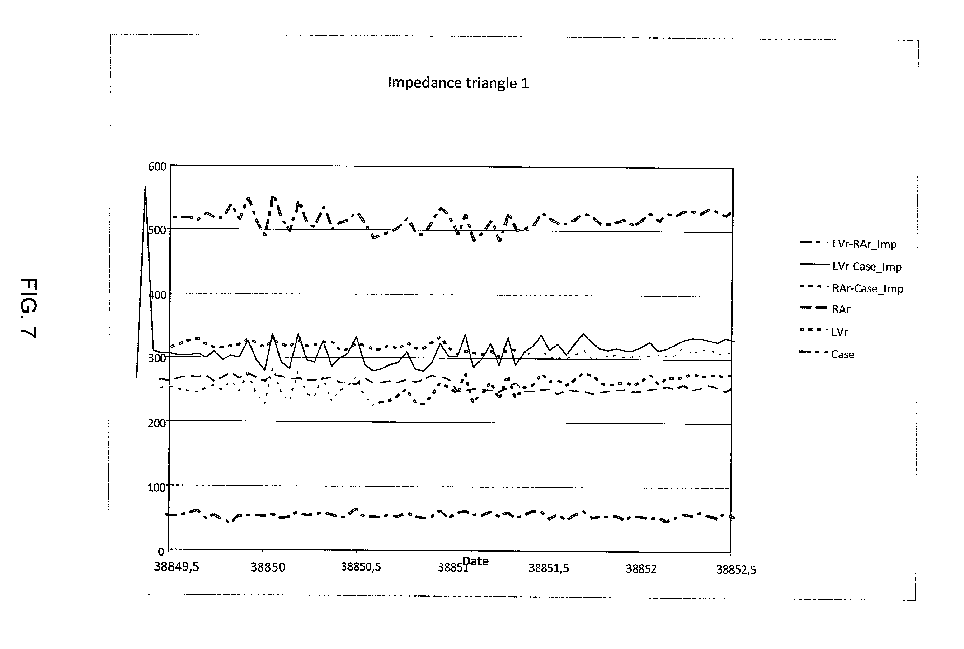

A measurement session, during which the immittance measurements are performed, has preferably a duration of some seconds, at least one respiration cycle or a number of heart cycles, and is performed at regular intervals, e.g. once every hour or every two hours. By storing the determined atrial dilatation values in the memory unit it will be possible to identify both short-term and long-term changes. The graphs illustrated in figures 7 and 8 show impedance values during three days and may be regarded to show short-term changes. Long-term changes may be identified during time periods of weeks, months or even years.

In one embodiment one atrial electrode is an RAring electrode. Figures 4 and 5 show an RAring electrode arranged in the right atrium. The immittance measurement of the fluid volume surrounding the RAring will provide a measure of dilatation of the right atrium.

In another embodiment one atrial electrode is an LAring electrode and the immittance measurement of the fluid volume surrounding the LAring will provide a measure of dilatation of the left atrium. This is also illustrated in figure 4.

As discussed above at least three electrodes are required to perform the immittance measurements.

One specific embodiment of the present invention is achieved when the immittance measurements are made between electrodes that correspond to an impedance triangle, i.e. when the immittance measurements are performed with measurement nodes arranged in a triangle.

In all cases, conversion of the immittance measurement values into relative near-field immittance values is achieved, by the immittance converter, by ignoring far-field contributions.

This is achieved, as discussed above, by converting the immittance measurement values into near-field immittance values, by the immittance converter, by converting at least N immittance measurement values (v1, v2, ..., vN) into a set of linear equations to be solved while ignoring the far-field contributions to the immittance measurements, where N is at least three; and by solving the set of linear equations to yield a set of near-field immittance values (e1, e2, ..., eN).

Thus, the immittance converter is adapted to convert the immittance measurement values into relative near-field immittance values by ignoring far-field contributions. More specifically, the immittance converter is adapted to convert the immittance measurement values into near-field immittance values by converting at least N immittance measurement values (v1, v2, ..., vN) into a set of linear equations to be solved while ignoring the far-field contributions to the immittance measurements, where N is at least three, and by solving the set of linear equations to yield a set of near-field immittance values (e1, e2, ..., eN).

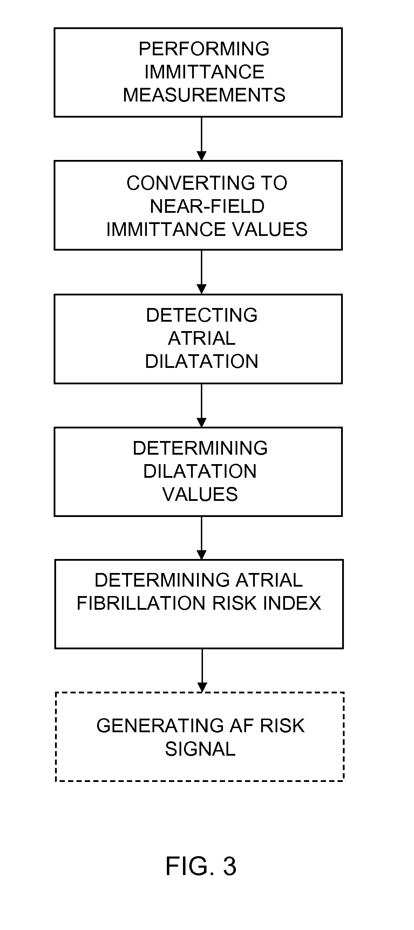

With reference to figure 3, the present invention also relates to a method for use in an implantable medical device for implantation within a patient.
The method comprises:
- performing immittance measurements within the heart of the patient using at least three electrodes connected to the device, where at least one electrode is arranged within an atrium of the patient;
- converting the immittance measurement values to individual near-field immittance values for at least one of said at least one electrode arranged within an atrium;
- detecting atrial dilatation based upon the near-field immittance values, and determining atrial dilatation values in dependence thereto, and
- determining an atrial fibrillation risk index based upon said atrial dilatation values.

In the figure is also included, as an optional step, that the method includes generating an AF risk signal in dependence of said risk index.

The method may further include comparing the determined atrial dilatation values with at least one atrial fibrillation risk threshold and generating the atrial fibrillation risk index in dependence of the comparison. The determined atrial fibrillation risk index is based upon the variations of the determined atrial dilatation values during a preset time period, which is discussed in detail above.

Preferably, one of the at least one atrial electrode is an RAring electrode and the immittance measurement of the fluid volume surrounding the RAring will provide a measure of atrial dilatation.

In another embodiment one of the at least one atrial electrode is an LAring electrode and the immittance measurement of the fluid volume surrounding the LAring will provide a measure of atrial dilatation.

One specific embodiment of the present invention is achieved when the immittance measurements are made between electrodes that correspond to an impedance triangle, i.e. when the immittance measurements are performed with measurement nodes arranged in a triangle.

The conversion of the immittance measurement values into relative near-field immittance values is achieved by ignoring far-field contributions to the impedance measurements, which specifically is achieved by:
converting at least N immittance measurement values (v1, v2, ..., vN) into a set of linear equations to be solved while ignoring the far-field contributions to the immittance measurements, where N is at least three, and solving the set of linear equations to yield a set of near-field immittance values (e1, e2, ... ,eN).

The method preferably includes the step of controlling at least one device function in response to the near-field immittance values. This may be a specifically tailored stimulation mode adapted to reduce the effect of AF, or even to prevent the occurrence of AF.

Figures 4 and 5 illustrate two different electrode set-ups which both would be applicable in relation to the present invention. A lead located in the left atrium, as well as in the right, would provide near-field impedance from that specific location and the near-field impedance signals from both atria can be monitored. This would indicate where the dilatation and therefore the fibrotic tissue reside. The fibrosis of tissue is a consequence of the atrial remodelling, which, in turn, is caused by the activation of hormone systems as a response to the atrial dilatation. The fibrotic tissue might be the substrate for AF and subsequently the origin of AF. A dilatation originating in the RA may be indicative of a right-sided disease, such as pulmonic or tricuspid valve stenosis, pulmonary disease or chronic obstructive pulmonary disease (COPD). A dilatation originating in the LA may be indicative of e.g. aortic or mitralis valve stenosis or systemic hypertension. The invention could thus provide an improved monitoring of disease progression and dilatation/AF.

Figure 6 shows a graph illustrating impedance signals from a healthy heart chamber (upper curve) that will have a higher DC level and larger peak-to-peak amplitude than a signal originating from a dilated chamber (lower curve).

Figure 7 shows impedance signals from a pre-clinical study. The upper lines are the recorded signals from three Z configurations in an impedance triangle 1. By references to figure 4 the impedance triangle is formed between left ventricle ring electrode (LVr) which could be one of LVring1, LVring2 or LVring3 in figure 4, right atrial ring electrode (RAr) which is denoted RAring in figure 4, and the case. The three lower lines are the near-field signals for the respective electrode extracted through the equation system outlined above.

According to one embodiment the relationship between different anatomical regions in the heart may be reflected by the signals obtained from one or several impedance triangles. For instance, by comparing the different terms (electrode nodes) in one impedance triangle, or the relation between electrode nodes from several impedance triangles, e.g. the configuration RAring-LVring included in the impedance triangle referred to in relation to figure 7, and another impedance triangle formed e.g. by the electrodes RAring, LVring3 and the case (can) by references to figures 4 or 5. The two different RAring signals will be extracted from two equation systems formed by the two impedance triangles and these signals will reflect the near-field in the RA produced by two different configurations.

The present invention is not limited to the above-described preferred embodiments. Various alternatives, modifications and equivalents may be used. Therefore, the above embodiments should not be taken as limiting the scope of the invention, which is defined by the appending claims.

## Claims

1. An implantable medical device connectable to at least three electrodes, said implantable medical device comprising:
an immittance measurer operative to perform immittance measurements within the heart of a patient using at least three of said electrodes whereby at least one of said electrodes is arranged in an atrium of the patient's heart; wherein
the immittance measurer is operative to perform said immittance measurements with electrodes as measurement nodes arranged in a triangle to form a respective immittance measurement value for each pair of measurement nodes, the medical device further comprises:
an immittance converter operative to convert said immittance measurement values into individual near-field immittance values of at least one of said at least one electrode being arranged in an atrium;
an atrial dilatation detector operative to detect atrial dilatation based upon said individual near-field immittance values, and to determine atrial dilatation values in dependence thereto, and
an atrial fibrillation risk determiner adapted to determine an atrial fibrillation risk index based upon said atrial dilatation values, .

2. The implantable medical device according to claim 1, wherein said atrial fibrillation risk determiner is adapted to generate an atrial fibrillation risk signal in dependence of said risk index.

3. The implantable medical device according to claim 1 or 2, wherein the atrial dilatation detector comprises a memory unit for storage of said determined atrial dilatation values.

4. The implantable medical device according to any preceding claim, wherein said atrial fibrillation risk determiner comprises a comparison unit provided with at least one atrial fibrillation risk threshold, wherein said comparison unit is adapted to compare said determined atrial dilatation values with said at least one atrial fibrillation risk threshold and wherein said atrial fibrillation risk determiner is adapted to determine said atrial fibrillation risk index in dependence of the comparison.

5. The implantable medical device according to any of claims 1-4, wherein the determined atrial fibrillation risk index is based upon the variations of the determined atrial dilatation values during a preset time period.

6. The implantable medical device according to any of claims 1-5, wherein one of said at least one atrial electrode is a right atrial ring electrode.

7. The implantable medical device according to any of claims 1-6, wherein one of said at least one atrial electrode is a left atrial ring electrode.
wherein said immittance converter is adapted to convert the immittance measurement values into near-field immittance values by converting at least N immittance measurement values (v1, v2, ..., vN) into a set of linear equations to be solved
where N is at least three and by solving the set of linear equations to yield a set of near-field immittance values (e1, e2, ..., eN),
wherein the equations have the form
vk = ep + eq (for k=1..N),
where p and q are the nodes of the pair of nodes for which the immittance measurement value was obtained.

## Patentansprüche

1. Implantierbares Medizinprodukt, das mit mindestens drei Elektroden verbindbar ist, wobei das implantierbare Medizinprodukt Folgendes umfasst:
eine Immittanz-Messeinrichtung, die so funktioniert, dass sie Immittanz-Messungen im Herzen eines Patienten unter Verwendung von mindestens drei der Elektroden durchführt, wobei mindestens eine der Elektroden in einem Vorhof des Herzen des Patienten angeordnet ist; wobei
die Immittanz-Messeinrichtung so funktioniert, dass sie die Immittanz-Messungen mit Elektroden als in einem Dreieck angeordneten Messknoten durchführt und so einen jeweiligen Immittanz-Messwert für jedes Paar von Messknoten bildet, wobei das Medizinprodukt ferner Folgendes umfasst:
einen Immittanz-Wandler, der so funktioniert, dass er die Immittanz-Messwerte in einzelne Nahfeld-Immittanz-Werte von mindestens einer der mindestens einen Elektrode umwandelt, die in einem Vorhof angeordnet ist;
eine Nachweiseinrichtung für eine Vorhofvergrößerung, die so funktioniert, dass sie eine Vorhofvergrößerung auf der Grundlage der einzelnen Nahfeld-Immittanz-Werte erkennt und abhängig davon Vorhofvergrößerungswerte bestimmt, und
eine Einrichtung zum Bestimmen der Gefahr eines Vorhofflimmerns, die so ausgelegt ist, dass sie auf der Grundlage der Vorhofvergrößerungswerte einen Vorhofflimmern-Gefahrenindex bestimmt,
wobei der Immittanz-Wandler so ausgelegt ist, dass er die Immittanz-Messwerte in Nahfeld-Immittanz-Werte umwandelt, indem er mindestens N Immittanz-Messwerte (v1, v2, ..., vN) in eine Menge von zu lösenden linearen Gleichungen umwandelt, wobei N mindestens drei ist, und
die Menge linearer Gleichungen löst und so eine Menge von Nahfeld-Immittanz-Werten (e1, e2, ..., eN) erhält, wobei die Gleichungen die Form vk = ep + eq aufweisen (für k=1..N),
wobei p und q die Knoten des Paars von Knoten sind, für die der Immittanz-Messwert erhalten wurde.

2. Implantierbares Medizinprodukt nach Anspruch 1, bei dem die Einrichtung zum Bestimmen der Gefahr eines Vorhofflimmerns so ausgelegt ist, dass sie ein Signal für die Gefahr eines Vorhofflimmerns in Abhängigkeit von dem Gefahrenindex erzeugt.

3. Implantierbares Medizinprodukt nach Anspruch 1 oder 2, wobei die Nachweiseinrichtung für eine Vorhofvergrößerung eine Speichereinheit zum Speichern der bestimmten Vorhofvergrößerungswerte umfasst.

4. Implantierbares Medizinprodukt nach einem vorhergehenden Anspruch, wobei die Einrichtung zum Bestimmen der Gefahr eines Vorhofflimmerns eine Vergleichseinheit umfasst, die mit mindestens einem Schwellenwert für die Gefahr eines Vorhofflimmerns versehen ist, wobei die Vergleichseinheit so ausgelegt ist, dass sie die bestimmten Vorhofvergrößerungswerte mit dem mindestens einen Schwellenwert für die Gefahr eines Vorhofflimmerns vergleicht und wobei die Einrichtung zum Bestimmen der Gefahr eines Vorhofflimmerns so ausgelegt ist, dass sie den Vorhofflimmern-Gefahrenindex in Abhängigkeit von dem Vergleich bestimmt.

5. Implantierbares Medizinprodukt nach einem der Ansprüche 1 bis 4, wobei der bestimmte Vorhofflimmern-Gefahrenindex auf den Veränderungen der bestimmten Vorhofvergrößerungswerte während einem vorher festgelegten Zeitraum beruht.

6. Implantierbares Medizinprodukt nach einem der Ansprüche 1 bis 5, wobei eine von der mindestens einen Vorhofelektrode eine Ringelektrode des rechten Vorhofs ist.

7. Implantierbares Medizinprodukt nach einem der Ansprüche 1 bis 6, wobei eine von der mindestens einen Vorhofelektrode eine Ringelektrode des linken Vorhofs ist.

## Revendications

1. Dispositif médical implantable raccordable à au moins trois électrodes, ledit dispositif médical implantable comprenant :
un mesureur d'immittance permettant de réaliser des mesures de l'immittance dans le coeur d'un patient au moyen d'au moins trois desdites électrodes dont au moins une est agencée dans une oreillette du coeur du patient ; dans lequel
le mesureur d'immittance permet de réaliser lesdites mesures d'immittance au moyen des électrodes formant des noeuds de mesure agencés en triangle pour former une valeur de mesure d'immitance respective pour chaque paire de noeuds de mesure, le dispositif médical comprenant en outre :
un convertisseur d'immittance permettant de convertir lesdites valeurs de mesure d'immittance en valeurs d'immittance en champ proche individuelles d'au moins une desdites au moins une électrode agencée dans une oreillette ;
un détecteur de dilatation auriculaire permettant de détecter une dilatation auriculaire en fonction desdites valeurs d'immittance en champ proche individuelles, et de déterminer des valeurs de dilatation auriculaire en fonction de ces dernières, et
un déterminateur de risque de fibrillation auriculaire conçu pour déterminer un indice de risque de fibrillation auriculaire en fonction desdites valeurs de dilatation auriculaire ; ledit convertisseur d'immittance étant conçu pour convertir les valeurs de mesure d'immittance en des valeurs d'immittance en champ proche en convertissant au moins N valeurs de mesure d'immittance (v1, v2, ..., vN) en un ensemble d'équations linéaires à résoudre, dans lesquelles N vaut au moins trois et en résolvant l'ensemble d'équations linéaires pour produire un ensemble de valeurs d'immittance en champ proche (e1, e2, ..., eN), les équations ayant la forme
vk = ep + eq (pour k = 1..N),
où p et q sont les noeuds de la paire de noeuds pour lesquels la valeur de mesure d'immittance a été obtenue.

2. Dispositif médical implantable selon la revendication 1, dans lequel ledit déterminateur de risque de fibrillation auriculaire est conçu pour générer un signal de risque de fibrillation auriculaire en fonction dudit indice de risque.

3. Dispositif médical implantable selon la revendication 1 ou 2, dans lequel le détecteur de dilatation auriculaire comprend une unité de mémoire destinée au stockage desdites valeurs de dilatation auriculaire déterminées.

4. Dispositif médical implantable selon l'une quelconque des revendications précédentes, dans lequel ledit déterminateur de risque de fibrillation auriculaire comprend une unité de comparaison pourvue d'au moins un seuil de risque de fibrillation auriculaire, ladite unité de comparaison étant conçue pour comparer lesdites valeurs de dilatation auriculaire déterminées audit au moins un seuil de risque de fibrillation auriculaire, et ledit déterminateur de risque de fibrillation auriculaire étant conçu pour déterminer ledit indice de risque de fibrillation auriculaire en fonction de la comparaison.

5. Dispositif médical implantable selon l'une quelconque des revendications 1 à 4, dans lequel l'indice de risque de fibrillation auriculaire déterminé repose sur les variations des valeurs de dilatation auriculaire déterminées, pendant une période de temps prédéfinie.

6. Dispositif médical implantable selon l'une quelconque des revendications 1 à 5, dans lequel une desdites au moins une électrode auriculaire est une électrode annulaire auriculaire droite.

7. Dispositif médical implantable selon l'une quelconque des revendications 1 à 6, dans lequel une desdites au moins une électrode auriculaire est une électrode annulaire auriculaire gauche.
